# EUROPEAN PATENT SPECIFICATION

(11) **EP 2 431 073 B1**
(45) Date of publication and mention of the grant of the patent: **29.07.2015**
(21) Application number: 11194015.1
(22) Date of filing: 31.10.2007
(51) Int. Cl.: A61N 5/06, A61B 18/20

(54) **Disposable tip for laser handpiece**
Einwegspitze für ein Laserhandstück
Embout jetable pour pièce à main laser

(30) Priority: 31.10.2006 US 589799
(43) Date of publication of application: 21.03.2012
(62) Divisional of application: 07853064.9
(73) Proprietor: Lumenis Ltd., 20692 Yokneam (IL)
(72) Inventor: Ramstad, Paul, San Jose, CA 95129 (US); Zakowski, Albert C., San Jose, CA 95132 (US); Smithson, Stephen D., Redwood City, CA 94061 (US); Degtyaryov, Andrey, Sunnyvale, CA 94061 (US); Gluszczak, Michael, San Jose, CA 95123 (US); White, Raymond G., Saratoga, CA 95070 (US); Bader, Ronald S., Boulder Creek, CA 95006 (US); Childs, Daniel K., Chicago, IL 60607 (US)
(74) Representative: Virdee-Crofts, Kulwinder Kaur

(56) References cited:
- EP-A1- 1 627 662
- WO-A1-01/34048
- WO-A2-2006/031632
- US-A1- 2006 189 964

## Description

### Technical Field

The present invention relates to the treatment of skin by irradiation with laser under vacuum.

Laser hand pieces for applying electromagnetic radiation to a patient's skin for a wide variety of treatments have been long known. Patients may have many different types of skin disorders, some of which may be contagious. There is therefore a great need in the skin treatment field for devices which avoid infecting patients when the same device is used on many patients. Further, in the laser treatment field specifically, there is a great need for safety. In particular, a user of a laser treatment device does not want the laser to fire if there is some component of the device improperly installed. Having a laser misfire could cause injury to the user, the patient, or an assistant standing nearby. There is therefore a great need in the art for maintaining hygienic practices in the skin treatment field as well as providing a measure of safety to a patient. Accordingly, there is now provided with this invention an improved laser radiation treatment system which effectively overcomes the aforementioned difficulties and long standing problems inherent in skin treatment systems. These problems have been solved in a simple, convenient, and highly effective way by which to have a disposable hygiene shield installed into a laser radiation hand piece.

### Prior Art

US 2006/0189964 discloses a system for applying laser radiation to skin. The publication describes a laser hand piece including a laser radiation source. The hand piece supports a projecting tip in which a cavity is formed. A vacuum can be induced in the cavity while the tip is applied to a section (region) of skin. During the application laser radiation can be delivered to the skin section contained within the chamber. The tip may be formed of a membrane and may be disposable.

### Summary Of The Invention

US 2006/0189964 discloses a system for applying laser radiation to skin. The publication describes a laser hand piece including a laser radiation source. The hand piece supports a projecting tip in which a cavity is formed. A vacuum can be induced in the cavity while the tip is applied to a section (region) of skin. During the application laser radiation can be delivered to the skin section contained within the chamber. The tip may be formed of a membrane and may be disposable.

As will be appreciated by those persons skilled in the art, a major advantage provided by the present invention is to have a disposable hygienic shield in a laser hand piece in which proper installation is detected. Additional objects of the present invention will become apparent from the following description.

The method and apparatus of the present invention will be better understood by reference to the following detailed discussion of specific embodiments and the attached figures which illustrate and exemplify such embodiments.

### Brief Description Of The Drawings

A specific embodiment of the present invention will now be described with reference to the following drawings, wherein:
Figure 1 is a diagrammatic view of an embodiment of the insert of the present invention and an embodiment of the laser hand piece into which it is inserted.
Figure 2 is an orthogonal view of an embodiment of the insert of the present invention.
Figure 3 is another orthogonal view of an embodiment of the insert of the present invention showing filters and O-rings in an exploded view.
Figure 4 is a bottom view of an embodiment of the insert of the present invention.
Figure 5 is a side view of an embodiment of the insert of the present invention taken along axis A-A of Figure 3.
Figure 6 is a side view of an embodiment of the insert of the present invention taken along axis B-B of Figure 3.
Figure 7 is an electrical schematic of an embodiment of a portion of the handpiece of the present invention.

### DESCRIPTION OF THE PREFERRED EMBODIMENT

The following preferred embodiment as exemplified by the drawings is illustrative of the invention and is not intended to limit the invention as encompassed by the claims of this application. A device adapted for insertion into a laser handpiece is disclosed herein.

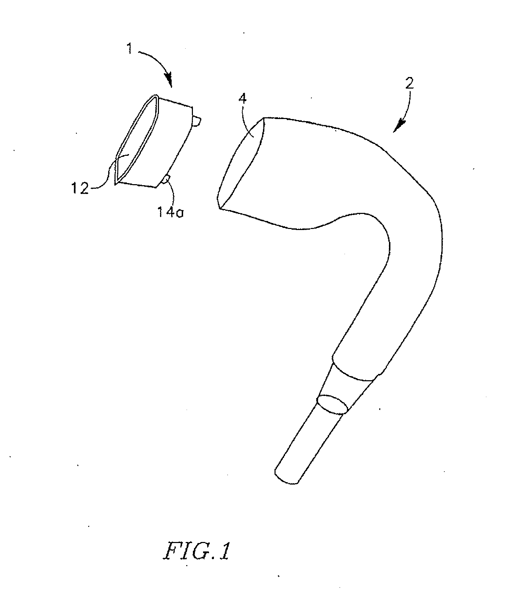

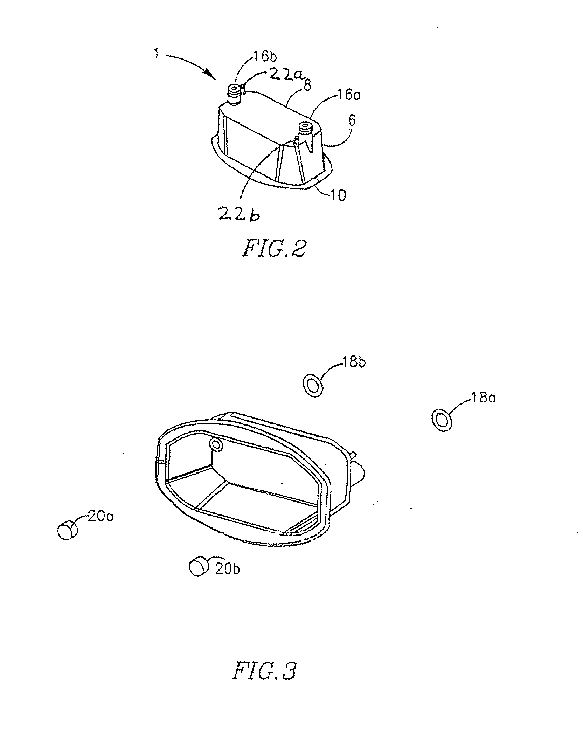

The insert 1, as illustrated generally in Figures 1 - 6 is for fitting into a laser handpiece 2. The insert acts, at least in part, as a hygiene shield for one time use on a patient. The laser handpiece has a cavity 4 into which the insert is configured for fitting. The cavity is adapted for applying a vacuum when the handpiece is applied to the skin for treatment. Laser light of particular wavelength and energy is directed to the skin while the skin is under vacuum with the handpiece in a predetermined controlled manner for selected skin treatment.

The insert 1 has an outer wall 6 which conforms to the cavity 4 of the handpiece. Typically, the thickness of the wall is in the range of from about 0.875 mm to about 1.125 mm. The insert has a base 8 which acts as a window through which the laser beam is directed. Typically, the thickness of the base is in the range of from about 0.875 mm to about 1.125 mm. Accordingly, at least the base is manufactured of a material optically transparent to the particular laser wavelength being applied. Preferably, the entire insert is made of the same material (for example, a polycarbonate having 88% light transmission and 1% haze at 2.5 mm) because laser light is reflected and redirected throughout the handpiece cavity by the walls of the cavity and thus also through the outer wall 6 of the insert. Preferably, the insert may be formed of clear GE Lexan HF 1140 Polycarbonate, FDA tripartite (ISO i0993-1 mod)/USP Class VI compliant, UL94V-2; or clear GE Lexan HP1, FDA food contact compliant, UL94V-2.

The base may also include optical properties either applied to or as part of its structure. Such optical properties may include, for example, a diffraction pattern, a refractive property, or an optical coating. Of course, as is well known to those skilled in the art, the base may be made of a different thickness or optical properties than the walls of the insert.

A further alternative to the body is to include either permanent or removable reflective surfaces along at least a portion of the side walls of the insert. The reflective surface, which may extend throughout the interior or exterior surfaces of the insert cavity and which may be either a coating or a removable insertion may be made of, or a substrate coated with, gold and/or other reflective metals and /or non-metals. The reflective surfaces may be specular, diffuse, or somewhere in between.

The insert due to its exposure to laser energy necessitates the use of a material with at least a Flame Class Rating of UL 94V-2 or (3) CSA. Since the insert comes in contact with the patient's skin, a biocompatible or FDA food contact compliant material is also needed. In addition to conforming plastics, glass is also a suitable material.

The outer wall extends from the base 8 to a peripheral flange 10 which extends around the periphery of the insert. The flange 10 is preferably conformable to the skin of the patient. It is preferable that the flange not extend beyond the handpiece body, except for a portion that is exposed to the side relief of the handpiece body for easing finger access for removal of the insert. Having the flange conform to the outer profile of the handpiece body allows the insert to remain in place when the handpiece is removed from a pocket into which it is preferably stored and calibrated. The outer wall thus forms a cavity 12 within the insert itself. The flange 10 is that portion of the insert that contacts the skin of the patient and forms a sufficient seal so that a vacuum, for example, ranging from about 11 in. Hg (37 kPa.) to about 28 in. Hg (95 kPa.), can be formed therewith. The vacuum thus formed preferably draws the skin into the cavity 12 of the insert before each pulse of electromagnetic radiation is applied and releases the skin upon completion of the pulse.

A channel 14a is formed into the insert for allowing vacuum communication between the handpiece and the cavity 12 of the insert. Preferably, such a channel is formed using a tubular member 16a extending from the base. It is further preferable that another similarly shaped member 16b be symmetrically formed on the base thereby forming another symmetrically placed channel 14b. It is still further preferable that only one of the two channels be operative at any one time. For example, although the insert mates exactly with the cavity 4 of the handpiece, and the two tubular members 16a and 16b fit into mating slots within the handpiece, only one of the members (for example, 16a) may be connected to a vacuum pump. Therefore, only one channel (for example, 14a) would allow passage for a vacuum to be applied to the cavity 12 of the insert when a seal is formed by the flange with the skin. The other tubular member (for example, 16b) would preferably fit into an inoperative channel of the handpiece (not shown). (Of course, as is well known in the art, both channels may alternatively be used for drawing a vacuum either simultaneously or in a sequence.)

In order to make a tight, leak-free sealing engagement with the vacuum channel of the handpiece, it may be preferable to include o-rings 18a and 18b on the tubular members 16a and 16b, respectively. The o-rings are preferably made of elastomeric material as is well known to those skilled in the art. It is further preferable that a 70-durometer silicone elastomer be used. Alternately, other durometers and other elastomers, including Buna-N, Viton, and other rubber equivalents may also be used.

The compression of the o-rings between the sealing surfaces of the handpiece and insert provides sufficient frictional resistance to secure the insert within the handpiece cavity during use. Other latching means such as mating protrusions and depressions integral to the insert and handpiece (or components within the handpiece) could also be used for this purpose. The body may also include a latching mechanism for securing the insert into the handpiece.

It may be preferable to include particle filters 20a and 20b inserted within the channels 14a and 14b, respectively. The preferred filter material is hydrophobic polyethylene, 3 mm thick, 45-90 micron pore size. Other suitable filter materials include polypropylene, Nylon, Teflon, glass, sintered metals and non-flammable grades of cellulose, preferably Porex X-4904 High Density Polyethylene meeting FDA 21 CFR 177.1520. Materials in different thicknesses, such as a woven or matted fabric or a wire mesh, with other pore sizes may also be acceptable for use in this application. Alternatively features such as a series of closely spaced protrusions or an array of small holes in a membrane-like element spanning the vacuum channel could be integrated into the design of the insert itself to replace the particle filters described in the current invention. The addition of the filters into their respective channels may help to protect the vacuum system from clogging with skin debris and other detritus that may be produced during the skin treatment procedure.

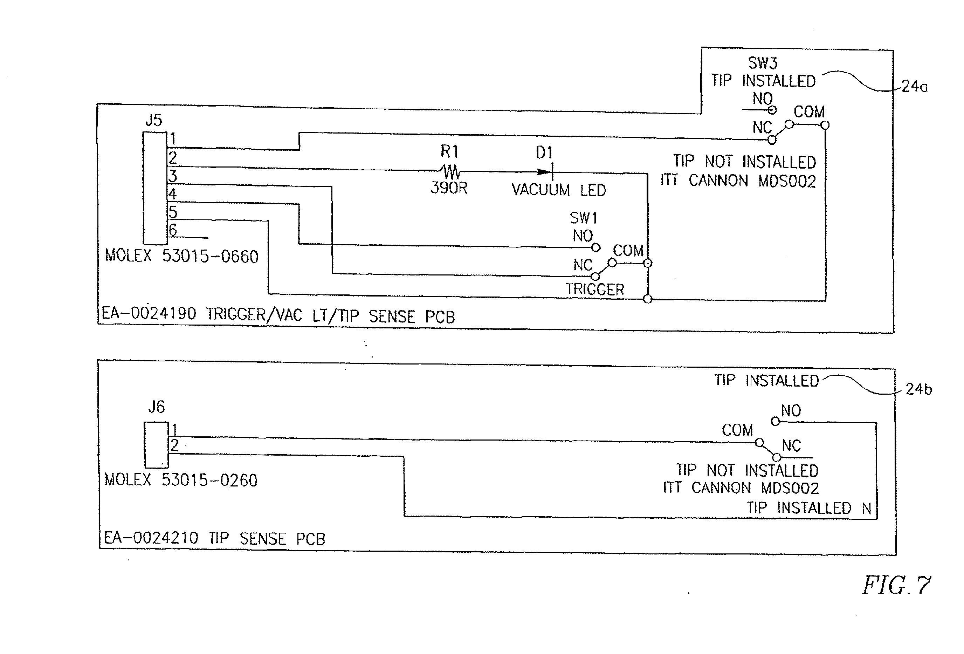

Tip pins 22a and 22b may be symmetrically placed extending from the base of the insert. These pins may be formed to matingly insert into pin receiving holes formed in the cavity of the handpiece. Within the pin receiving holes of the handpiece, which are formed to receive and engage with the tip pins 22a and 22b, are pin detectors for detecting the presence of both of the pins. The detection of the presence of both of the tip pins allows an additional level of safety for the operator and for the patient. Of course, as is well known in the art, alternatively, only one of the pin receiving holes may have a pin detector. A further alternative may exclude pins entirely and merely detect the presence of the tubular extensions 16a and 16b when they are inserted into their corresponding channels in the handpiece. Such detection of the tubular channels for conducting a vacuum would be in accordance with the electrical schematic diagram discussed with respect to Figure 7.

Figure 7 shows an electrical schematic diagram of an embodiment of pin detection. By providing this type of pin detection, a safety interlock with the laser is effected. As illustrated, one pin, for example 22a, may fit into a pin-receiving hole of the handpiece in which a normally closed detection switch is located. The other pin, for example, 22b, may correspondingly fit into another pin receiving hole of the handpiece in which a normally open detection switch is located. When the insert is properly secured into the handpiece, both of the pins must engage with their respective detection switches in order to allow the laser to become operative. In this particular embodiment described and illustrated in Figure 7, when pin 22a matingly engages with its receiving hole, a pin detection switch 24a is moved from its normally closed position to an open position. Correspondingly, when pin 22b matingly engages with its receiving hole, a pin detection switch 24b is moved from its normally open position to a closed position. When the circuit in which switch 24a is broken and when the circuit in which 24b is completed are both detected, the safety interlock allows the laser to fire. Only when both circuits are detected, with switch 24a being open and switch 24b being closed, is the laser allowed to fire. This arrangement is preferable because it ensures that misdetection will not result in allowing the laser to fire.

Alternatively, the detection switches may be differently arranged. For example, switches 24a and 24b may both be normally closed switches. As a further alternative, switches 24a and 24b may both be normally open switches.

A further alternative to having pins extending from the insert into receiving holes of the insert may be to have a pin or pins in the handpiece extend into receiving holes or receiving depressions in the insert. In such an embodiment, the safety interlock would determine whether or not the pins extend from the handpiece in the correct manner. In such an embodiment, only when the pins correctly extend from the handpiece into their respective corresponding receptors in the insert, would the laser be allowed to fire.

A still further embodiment of the present invention may include using a conductive material to complete a circuit between two electrodes in the handpiece cavity. All or a selected portion of the insert may be conductive. Thus, when the insert in properly placed in the handpiece, a circuit is completed for establishing proper fit. Alternatively, the pins 22a and 22b may be conducting elements or electrodes.

A yet further embodiment of the present invention may include a capacitive or optical proximity detector for non-contact sensing of the insert. An additional embodiment may further include a means for detecting an optical signature of the base. Such detection of the optical signature of the base may include, for example, detecting the reflection signature or the absorption signature of the base for an indication of correct insertion of the tip into the handpiece.

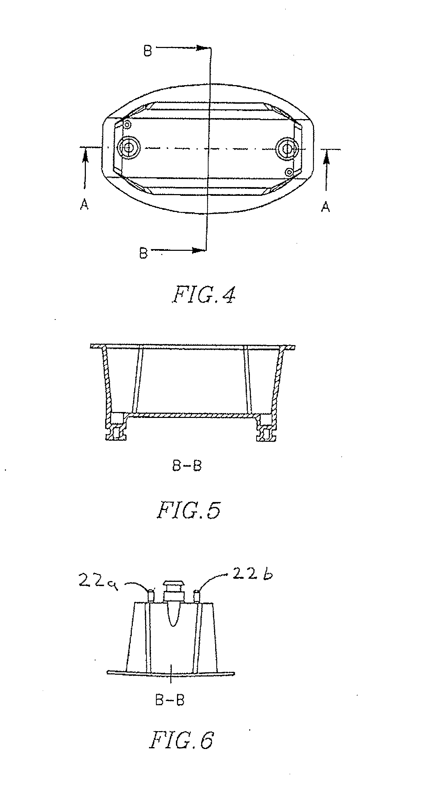

It is preferable that the insert be symmetrical about axes A-A and B-B of Figure 4. In this way, the insert can initially be placed in the cavity of the handpiece easily. Further, since an embodiment of the present invention may have only one of the two channels (for example, 14a) operative for drawing a vacuum, if one filter, for example 20a becomes clogged, the same insert can be easily and quickly repositioned in the handpiece thereby using the other channel (for example, 14b) with the unused filter, for example, 20b. After the insert has been used on a patient, it is disposed of so that the next patient may have a new hygienic insert installed for his use. Reuse of a cleaned sterilized insert is also within the scope of this invention.

Although the particular embodiments shown and described above will prove to be useful in many applications in the skin treatment arts and laser applications in general, to which the present invention pertains, further modifications of the present invention will occur to persons skilled in the art. All such modifications are deemed to be within the scope of the present invention as defined by the appended claims.

## Claims

1. A system for applying laser radiation to skin of a patient, the system comprising:
(i) a laser hand piece (2) having a first cavity (4) and including a laser radiation source for applying radiation into the first cavity (4), and a vacuum channel; **characterised in that** there is provided
(ii) a removable insert (1) having a base (8), and an outer wall (6) extending from the base (8) to peripheral flange (10), wherein :
the base (8) and/or outer wall (6) at least partially conform to the first cavity (4) whereby the insert (1) is adapted for inserting into the first cavity (4);
the base (8) and outer wall (6) defining a second cavity (12) within the removable insert (1);
the flange (10) is configured to form a seal against a section of skin;
wherein at leat the base (8) of the removable insert (1) is formed from a material transparent to a wavelength transmitted by the laser radiation source, and
wherein the removable insert (1) has at least one insert vacuum channel (14a,b) configured to provide vacuum communication between the second cavity (12) and the vacuum channel of the hand piece (2) to facilitate the establishment of a vacuum in the second cavity (12) when the flange (10) is sealingly applied to a section of skin tissue whereby the vacuum can cause the section of skin tissue to be drawn into the cavity (12).

2. The system of claim 1, further comprising at least one means for detecting proper insertion of the removable insert (1) into the first cavity (4) of the hand piece (2).

3. The system of claim 2, wherein means for detecting proper insertion includes at least one pin (22a,b) extending into the hand piece (2) so that at least one pin can be detected therein when said removable insert (1) is fitted into said hand piece (2).

4. The system of claim 2, the hand piece (2) further comprising a safety interlock coupled to the means for detecting proper insertion of the removable insert (1), wherein the safety interlock renders the radiation source inoperative when proper insertion of the removable insert (1) into the first cavity (4) is not detected.

5. The system of claim 1, wherein the removable insert (1) includes an optical element, the optical element including one or more of:
(i) a lens;
(ii) a diffraction grating;
(iii) a refractive element; and
(iv) an optical coating.

6. The system of claim 1, wherein the removable insert (1) includes one or more of:
(i) a reflecting surface; and
(ii) a latching mechanism.

7. The system of claim 1, wherein the at least one channel (14a,b) is adapted for including a particle filter (20a, b).

8. The system of claim 1, wherein the at least one channel (14a, b) comprises at least one member (16a, b) extending from the base (8) into the handset (2).

9. The system of any one of the preceding claims wherein the flange (10) is conformable to the skin section.

## Patentansprüche

1. System zum Anwenden von Laserstrahlung auf Haut eines Patienten, wobei das System Folgendes umfasst:
(i) ein Laserhandteil (2), das einen ersten Hohlraum (4) aufweist und eine Laserstrahlungsquelle zum Anwenden von Strahlung in den ersten Hohlraum (4) und einen Vakuumkanal beinhaltet;
**dadurch gekennzeichnet, dass** Folgendes vorgesehen ist:
(ii) ein herausnehmbarer Einsatz (1), der eine Basis (8) und eine Außenwand (6) aufweist, die sich von der Basis (8) erstreckt, um einen Umfangsflansch (10) zu tragen, wobei:
die Basis (8) und/oder die Außenwand (6) zumindest zum Teil dem ersten Hohlraum (4) entspricht, wodurch der Einsatz (1) zum Einsetzen in den ersten Hohlraum (4) angepasst ist;
die Basis (8) und die Außenwand (6) einen zweiten Hohlraum (12) in dem herausnehmbaren Einsatz (1) definieren;
der Flansch (10) dazu konfiguriert ist, eine Abdichtung gegen einen Hautabschnitt zu bilden;
wobei mindestens die Basis (8) des herausnehmbaren Einsatzes (1) aus einem Material hergestellt ist, das für eine Wellenlänge durchlässig ist, die von der Laserstrahlungsquelle übertragen wird, und
wobei der herausnehmbare Einsatz (1) mindestens einen Einsatzvakuumkanal (14a, 14b) aufweist, der dazu konfiguriert ist, eine Vakuumverbindung zwischen dem zweiten Hohlraum (12) und dem Vakuumkanal des Handteils (2) bereitzustellen, um die Herstellung eines Vakuums in dem zweiten Hohlraum (12) zu erleichtern, wenn der Flansch (10) abdichtend auf einen Hautgewebeabschnitt aufgebracht wird, wodurch das Vakuum bewirken kann, dass der Hautgewebeabschnitt in den Hohlraum (12) gezogen wird.

2. System nach Anspruch 1, das weiterhin mindestens ein Mittel zum Erkennen eines korrekten Einsetzens des herausnehmbaren Einsatzes (1) in den ersten Hohlraum (4) des Handteils (2) umfasst.

3. System nach Anspruch 2, wobei das Mittel zum Erkennen eines korrekten Einsetzens mindestens einen Stift (22a, 22b) beinhaltet, der sich in das Handteil (2) erstreckt, so dass mindestens ein Stift darin erkannt werden kann, wenn der herausnehmbare Einsatz (1) in das Handteil (2) eingepasst wird.

4. System nach Anspruch 2, wobei das Handteil (2) weiterhin eine Sicherheitssperre umfasst, die mit dem Mittel zum Erkennen eines korrekten Einsetzens des herausnehmbaren Einsatzes (1) verbunden ist, wobei die Sicherheitssperre die Strahlungsquelle funktionsunfähig macht, wenn ein korrektes Einsetzen des herausnehmbaren Einsatzes (1) in den ersten Hohlraum (4) nicht erkannt wird.

5. System nach Anspruch 1, wobei der herausnehmbare Einsatz (1) ein optisches Element beinhaltet, wobei das optische Element eines oder mehrere der folgenden beinhaltet:
(i) eine Linse;
(ii) ein Beugungsgitter;
(ii) ein Brechungselement und
(iv) eine optische Beschichtung.

6. System nach Anspruch 1, wobei der herausnehmbare Einsatz (1) eines oder mehrere der folgenden beinhaltet:
(i) eine reflektierende Oberfläche und
(ii) einen Arretierungsmechanismus.

7. System nach Anspruch 1, wobei der mindestens eine Kanal (14a, 14b) zum Beinhalten eines Partikelfilters (20a, 20b) angepasst ist.

8. System nach Anspruch 1, wobei der mindestens eine Kanal (14a, 14b) mindestens ein Element (16a, 16b) umfasst, das sich von der Basis (8) in das Handteil (2) erstreckt.

9. System nach einem der vorhergehenden Ansprüche, wobei der Flansch (10) an den Hautabschnitt anpassungsfähig ist.

## Revendications

1. Système pour appliquer un rayonnement laser sur la peau d'un patient, le système comprenant :
(i) une pièce à main laser (2) possédant une première cavité (4) et incluant une source de rayonnement laser pour appliquer un rayonnement dans la première cavité (4), et un canal à dépression ;
**caractérisé en ce que** sont prévues :
(ii) une pièce rapportée amovible (1) avec une base (8), et une paroi externe (6) laquelle se prolonge à partir de la base (8) jusqu'à une bride périphérique (10),
cas dans lequel :
la base (8) et/ou la paroi externe (6) suivent au moins partiellement le profil de la première cavité (4), de ce fait la pièce rapportée (1) est conçue en vue d'une insertion dans la première cavité (4) ;
la base (8) et la paroi externe (6) définissant une deuxième cavité (12) à l'intérieur de la pièce rapportée amovible (1) ;
la bride (10) est configurée de façon à former un joint étanche contre une section de peau ;
cas dans lequel au moins la base (8) de la pièce rapportée amovible (1) est réalisée à partir d'une matière qui est transparente à une longueur d'onde transmise par la source de rayonnement laser, et
cas dans lequel la pièce rapportée amovible (1) possède au moins un canal à dépression de pièce rapportée (14a, b) lequel est configuré de façon à procurer une communication à dépression entre la deuxième cavité (12) et le canal à dépression de la pièce à main (2) afin de faciliter l'établissement d'une dépression dans la deuxième cavité (12) lorsque la bride (10) est appliquée de façon étanchéisante à une section de tissu cutané, de ce fait la dépression peut provoquer l'aspiration de la section de tissu cutané jusque dans la cavité (12).

2. Système selon la revendication 1, comprenant en outre au moins un moyen pour détecter l'insertion correcte de la pièce rapportée amovible (1) dans la première cavité (4) de la pièce à main (2).

3. Système selon la revendication 2, le moyen pour détecter l'insertion correcte incluant au moins une broche (22a, b) laquelle se prolonge jusque dans la pièce à main (2) de sorte qu'au moins une broche puisse être détectée dans celle-ci lorsque ladite pièce rapportée amovible (1) est montée dans ladite pièce à main (2).

4. Système selon la revendication 2, la pièce à main (2) comprenant en outre un verrouillage de sécurité couplé au moyen pour détecter l'insertion correcte de la pièce rapportée amovible (1), cas dans lequel le verrouillage de sécurité rend inopérante la source de rayonnement lorsque l'insertion correcte de la pièce rapportée amovible (1) dans la première cavité (4) n'est pas détectée.

5. Système selon la revendication 1, la pièce rapportée amovible (1) incluant un élément optique, l'élément optique incluant un ou plusieurs des postes suivants :
(i) une lentille ;
(ii) une grille de diffraction ;
(iii) un élément de réfraction ; et
(iv) un revêtement optique.

6. Système selon la revendication 1, la pièce rapportée amovible (1) incluant un ou plusieurs des postes suivants :
(i) une surface réfléchissante ; et
(ii) un mécanisme à enclenchement.

7. Système selon la revendication 1, ledit au moins un canal (14a, b) étant conçu pour inclure un filtre à particules (20a, b).

8. Système selon la revendication 1, ledit au moins un canal (14a, b) comprenant au moins un élément (16a, b) lequel se prolonge à partir de la base (8) jusque dans l'appareil portatif (2).

9. Système selon l'une quelconque des revendications précédentes, la bride (10) étant apte à suivre le profil de la section de peau.
